# EUROPÄISCHE PATENTSCHRIFT

(11) **EP 4 404 872 B1**
(45) Veröffentlichungstag und Bekanntmachung des Hinweises auf die Patenterteilung: **25.02.2026**
(21) Anmeldenummer: 22786799.1
(22) Anmeldetag: 22.09.2022
(51) Int. Cl.: A61F 2/16

(54) **OPHTHALMOLOGISCHES IMPLANTAT MIT UNTERSCHIEDLICH STEIFEN OBERFLÄCHENBEREICHEN**
OPTHALMOLOGICAL IMPLANT HAVING SURFACE REGIONS WITH DIFFERENT STIFFNESSES
IMPLANT OPHTALMOLOGIQUE AYANT DES RÉGIONS DE SURFACE DE DIFFÉRENTES RIGIDITÉS

(30) Priorität: 22.09.2021 DE 102021124516
(43) Veröffentlichungstag der Anmeldung: 31.07.2024
(73) Patentinhaber: Carl Zeiss Meditec AG, 07745 Jena (DE)
(72) Erfinder: NICOLI, Francesca, 10551 Berlin (DE); MASCH, Jennifer-Magdalena, 23909 Ratzeburg (DE)
(74) Vertreter: Hofstetter, Schurack & Partner
(86) Internationale Anmeldenummer: PCT/EP2022/076314
(87) Internationale Veröffentlichungsnummer: WO 2023/046811

(56) Entgegenhaltungen:
- US-A- 4 764 169
- US-A- 5 326 506
- US-A- 5 507 805
- US-A1- 2015 182 330
- US-B1- 6 391 230
- US-B2- 10 682 225
- US-B2- 8 216 310
- US-B2- 9 554 893

## Beschreibung

### Technisches Gebiet

Die Erfindung betrifft ein ophthalmologisches Implantat mit unterschiedlich steifen Oberflächenbereichen.

### Stand der Technik

Bei der Kataraktoperation wird die natürliche menschliche Linse durch eine künstliche intraokulare Linse (IOL) ersetzt. Die IOL wird im Äquator des menschlichen Kapselsacks platziert. Dabei wird die vordere Seite des Kapselsacks durch einen kreisförmigen Schnitt geöffnet. Die menschliche Linse wird durch das Loch, die Kapsulorhexis, entfernt. Diese Kreiswunde bleibt nach der Operation offen. Im Kapselsack verbleiben typischerweise Gewebereste, aus denen Zellen zur Wundheilung gebildet werden. Diese Zellen wandern entlang der Innenseite des Kapselsacks. Hierdurch kann nach Kataraktoperationen in manchen Fällen eine sogenannte posteriore Kapselopazifikation (posterior capsule opacification, PCO, Cataracta secundaria) auftreten.

Die posteriore Kapseltrübung (PCO) ist die häufigste postoperative Komplikation nach einer Kataraktoperation. Sie besteht in der Eintrübung des hinteren Kapselsacks (capsular bag, CB) aufgrund der Proliferation und Migration von Linsenepithelzellen (LECs) nach der Phakoemulsifikation. Diese Pathologie tritt bei etwa 20 bis 50 % der Patienten zwei bis fünf Jahre nach der Kataraktoperation auf und beeinträchtigt, wenn sie nicht behandelt wird, das Sehvermögen des Patienten. Die derzeit gängige Behandlung für PCO ist ein Verfahren, das Neodym:YAG (Nd:YAG)-Laser-Kapsulotomie genannt wird. Bei dieser Behandlung wird durch gepulste Laserstrahlung eine Öffnung von mehreren Millimetern im hinteren Teil der Kapselsacks geschaffen. Obwohl dieses Verfahren im Allgemeinen sicher ist, kann es in seltenen Fällen Nebenwirkungen haben. Ziel ist es daher generell, die PCO-Bildung nach einer Kataraktoperation möglichst gänzlich zu verhindern. Dieses Ziel der PCO-Prävention wird derzeit überwiegend auf zwei Wegen verfolgt, nämlich durch gezielte Variierung der Geometrie des Grundkörpers des Implantats und durch die Verabreichung und Freisetzung von Medikamenten.

Ein präventiver Schritt zur Verhinderung von PCO wird in der Gestaltung der Geometrie von IOLs gesehen. Tatsächlich zeigt sich, dass die Implantation von IOLs mit einem quaderförmigen Randdesign (Square-Edge-Design) im Vergleich zu abgerundeten Designs weniger anfällig für die Bildung von PCO ist. Es wird vermutet, dass dieser Effekt darauf zurückzuführen ist, dass die Quaderkanten eine Barriere bilden, über die Zellen nur schwer hinweg wandern können. Der zweite gängige Ansatz zur PCO-Prävention ist die pharmakologische Behandlung, bei der verschiedene Arten von Medikamenten getestet wurden, wie z.B. entzündungshemmende, proliferationshemmende und Apoptoseinduzierende Medikamente. Trotz des Square-Edge-Designs von IOLs und vieler getesteter Medikamente ist die PCO-Prävention weiterhin leider nicht hochwirksam, so dass die Hauptbehandlung für betroffene Patienten immer noch die Nd:YAG-Kapsulotomie ist.

US 10 682 225 B2 offenbart ein Verfahren und ein System zum Herstellen einer ophthalmischen Vorrichtung mit einer Optik und einer Haptik. Die Optik enthält mindestens ein optisches Material mit einem ersten Elastizitätsmodul. Die Haptik ist mit der Optik gekoppelt. Die Haptik weist mindestens einen zweiten Elastizitätsmodul auf, der größer als der erste Elastizitätsmodul und kleiner als 1,8 GPa ist. Somit ist die Haptik steifer als die Optik, aber flexibler als ein Material wie Polymethylmetacrylat (PMMA).

US 2015/182330 A1 offenbart ein intraokulares Implantat mit Eingabe- und/oder Ausgabeelektronik. In einigen Ausführungsformen umfasst das System eine intraokulare Linse mit mindestens einer Optik, die funktionsfähig mit einer Haptik gekoppelt ist, eine oder mehrere Eingabeelektronik(en) an der Haptik und/oder der Optik und eine oder mehrere Ausgabeelektronik(en) an der Haptik und/oder der Optik zum Empfangen und/oder Übertragen von Daten.

US 9 554 893 B2 offenbart eine Haptik zur Verwendung in einer akkommodierenden Intraokularlinse. Die Haptik hat mehrere Fäden, die jeweils an einem Ende mit dem Rand der Optik verbunden sind. Jeder Faden hat eine Form, die sich an einen äquatorialen Bereich des Kapselsacks anpasst. Die Haptik koppelt die Kräfte, die der Kapselsack des Auges während der Akkommodation ausübt, radial an den Rand der Optik und erzeugt eine diametrale Ausdehnung oder Kompression der Optik. Diese diametrale Bewegung führt zu einer Verformung der Linse, wodurch sich der vordere Radius, der hintere Radius und die Dicke ganz oder teilweise verändern.

US 8 216 310 B2 offenbart ein polymeres Material mit einer molekularen Reaktionszeit, die es für die Verwendung in der Nähe von empfindlichem Körpergewebe geeignet macht. Das polymere Material eignet sich sowohl für Anwendungen mit niedrigem Modul als auch mit hohem Modul, wodurch das Herstellungsverfahren für mehrteilige polymere Artikel vereinfacht und besser integrierte mehrteilige polymere Artikel geschaffen werden.

### Darstellung der Erfindung

Aufgabe der vorliegenden Erfindung ist es, ein ophthalmologisches Implantat zu schaffen, welches das PCO-Risiko verringert.

Die Aufgabe wird erfindungsgemäß durch ein ophthalmologisches Implantat gemäß Patentanspruch 1 gelöst. Vorteilhafte Ausgestaltungen mit zweckmäßigen Ausbildungen der Erfindung sind in den Unteransprüchen angegeben.

Ein erster Aspekt der Erfindung betrifft ein ophthalmologisches Implantat gemäß Anspruch 1 mit einem Grundkörper, welcher zumindest einen ersten Oberflächenbereich mit einem ersten Young'schen Modul und einen zweiten Oberflächenbereich mit einem zweiten Young'schen Modul umfasst, wobei sich der erste und der zweite Young'scher Modul voneinander unterscheiden. Mit anderen Worten ist es vorgesehen, dass das erfindungsgemäße ophthalmologische Implantat einen Grundkörper mit zwei oder mehr Oberflächenbereichen aufweist, die eine unterschiedliche Steifigkeit bzw. Elastizität, ausgedrückt durch den Young'schen Modul (Elastizitätsmodul, E-Modul), aufweisen. Der Young'sche Modul ist ein Materialkennwert, der den Zusammenhang zwischen Spannung und Dehnung bei der Verformung eines festen Körpers beschreibt. Vorzugsweise werden der erste und der zweite Young'sche Modul bei Körperkerntemperatur (37 °C) ermittelt. Bei dem erfindungsgemäßen Implantat ist also die Steifigkeit bzw. Elastizität des Grundkörpers zumindest an der mit Zellen in Kontakt bringbaren Oberfläche deterministisch auf (nur) zwei (oder mehr) Oberflächenbereiche mit unterschiedlichen E-Moduln eingestellt, um die letztlich nicht vermeidbare Zellmigration zu kontrollieren. Grundsätzlich können auch drei, vier, fünf oder mehr Oberflächenbereiche vorgesehen sein, wobei wenigstens zwei der Oberflächenbereiche und vorzugsweise alle Oberflächenbereiche jeweils unterschiedliche Young'sche Moduln besitzen.

Die Erfindung beruht dabei auf der Erkenntnis, dass der Grad der Steifigkeit bzw. Elastizität des Implantats das Zellverhalten beeinflusst. Insbesondere die Adhäsionspunkte zwischen den Zellen und der als Substrat fungierenden Oberfläche des Grundkörpers des Implantats sind stark von den Oberflächeneigenschaften abhängig, so dass sich das Zytoskelett, das für die Zellbewegung verantwortlich ist, auf einer weichen bzw. elastischen oder auf einer steifen Oberfläche unterschiedlich ausbreitet, was zu einer unterschiedlichen Zelldynamik führt. Das unterschiedliche Zelladhäsionsverhalten beeinflusst je nach Zelltyp die Migrationsgeschwindigkeit, die durch steifere oder weichere bzw. elastischere Substrate gezielt lokal gefördert oder gehemmt werden kann. Darüber hinaus können Zellen durch die Einstellung unterschiedlich steifer Oberflächenbereiche sogar gelenkt und damit gezielt auf einen gewünschten Bereich des Grundkörpers geleitet bzw. auf diesem Bereich konzentriert werden. Die mechanischen Eigenschaften des Grundkörpers des erfindungsgemäßen Implantats sind damit derart modifiziert, dass die Migration von Zellen, die aufgrund ihres Zellwachstums zu einer Trübung des Kapselsacks führen könnten, gehemmt oder selektiv auf bestimmte Bereiche des Implantats gelenkt werden kann. Damit wird das PCO-Risiko erheblich verringert. Das erfindungsgemäße Implantat hat gegenüber einer Medikamentenfreisetzung zudem den Vorteil, dass die für eine Medikamentenzulassung notwendige Entwicklung und die damit verbundenen umfangreichen Tests entfallen. Außerdem ändert sich bei medikamentenbeladenen Implantaten die Rate der Medikamentenfreisetzung mit der Zeit und ist bestenfalls auf einige Jahre nach der Kataraktoperation beschränkt. Bei dem vorgeschlagenen mechanischen Ansatz mit unterschiedlich steifen Oberflächenbereichen ist der Prozess, der die Zellmigration hemmt und steuert, eine intrinsische Eigenschaft des Implantats, die sich im Laufe der Zeit nicht oder nur unwesentlich verändert. Das erfindungsgemäße ophthalmologische Implantat kann beispielsweise als Intraokularlinse oder Ring ausgebildet sein. Hierdurch können die vorstehend genannten Vorteile bezüglich der kontrollierten Zellmigration für unterschiedliche Implantattypen realisiert werden. Generell sind "ein/eine" im Rahmen dieser Offenbarung als unbestimmte Artikel zu lesen, also ohne ausdrücklich gegenteilige Angabe immer auch als "mindestens ein/mindestens eine". Umgekehrt können "ein/eine" auch als "nur ein/nur eine" verstanden werden. Der Begriff "umfassen" wird im Rahmen dieser Offenbarung so verstanden, dass neben genannten Merkmalen weitere Merkmale vorhanden sein können. Der Begriff "umfassen" schließt aber auch "bestehen aus" mit ein, das heißt dass neben genannten Merkmalen keine weiteren Merkmale vorhanden sein können.

Erfindungsgemäß ist vorgesehen, dass der Grundkörper wenigstens einen haptischen Teil und wenigstens einen optischen Teil umfasst. Vorzugsweise sind der erste und zweite Oberflächenbereich und ihre jeweiligen Moduln in diesem Fall so gewählt, dass die Migration von Zellen in Bereiche des optischen Teils gehemmt oder verhindert oder in Bereiche gelenkt wird, die den optischen Teil und damit das Sehvermögen des Patienten nicht beeinträchtigen. Insbesondere werden die Zellen bevorzugt in Richtung der Haptik gelenkt. Hierdurch wird zuverlässig verhindert, dass die Funktionalität des optischen Teils beeinträchtigt wird. Darüber hinaus besteht die Möglichkeit, den haptischen Teil als physikalische Barriere gegen Zellwanderung zu gestalten.

Weiterhin ist erfindungsgemäß vorgesehen, dass der optische Teil den ersten Oberflächenbereich und der haptische Teil den zweiten Oberflächenbereich umfasst. Mit anderen Worten ist es vorgesehen, dass sich der optische Teil und der haptische Teil zumindest oberflächlich hinsichtlich ihres Young'schen Moduls unterscheiden. Hierdurch sind die zwei oder mehr Oberflächenbereiche derart gewählt, dass Zellen auf dem haptischen Teil "gefangen" werden können bzw. sich nur entlang des haptischen Teils ausbreiten und nicht auf den optischen Teil wandern.

Weiterhin ist erfindungsgemäß vorgesehen, dass der Grundkörper zumindest überwiegend aus einem einzelnen Material besteht, das im ersten und/oder im zweiten Oberflächenbereich modifiziert, insbesondere unterschiedlich stark quervernetzt ist, um die unterschiedlichen Young'schen Moduln zu erzeugen. Mit anderen Worten ist es vorgesehen, dass der Grundkörper grundsätzlich aus einem einzigen Material besteht, dessen E-Modul durch entsprechende Behandlung zumindest oberflächlich lokal verändert ist, um den ersten und zweiten Oberflächenbereich auszubilden. Dies kann beispielsweise durch Bestrahlung und/oder durch einen unterschiedlichen Quervernetzungsgrad erfolgen. Vorzugsweise ist das Material ein Polymer, insbesondere ein substituiertes oder unsubstituiertes (Meth)Acrylatpolymer. Der Grundkörper kann bevorzugt zu mindestens 90 Gew.-%, insbesondere zu mindestens 95 Gew.-%, beispielsweise zu 96 Gew.-% bis 99 Gew.-% aus dem einzigen bzw. einheitlichen bzw. unmodifizierten Material bestehen. Der Rest kann dann entsprechend aus dem modifizierten Material bestehen.

Weitere Vorteile ergeben sich dadurch, dass der zweite Oberflächenbereich unmittelbar an den ersten Oberflächenbereich angrenzt. Hierdurch kann ein scharfer, stufenförmiger Elastizitätsmodulwechsel realisiert werden, um die Wanderung von Zellen über die Grenze zwischen den beiden Oberflächenbereiche zu be- oder verhindern. Alternativ oder zusätzlich ist es vorgesehen, dass der zweite Oberflächenbereich vom ersten Oberflächenbereich beabstandet angeordnet ist. Mit anderen Worten ist also ein gewisser Abstand A>0 zwischen dem ersten und zweiten Oberflächenbereich vorhanden. Dies kann beispielsweise sinnvoll sein, wenn das Implantat Zwischenräume zwischen den Oberflächenbereichen aufweisen soll. Alternativ oder zusätzlich ist es vorgesehen, dass der zweite Oberflächenbereich den ersten Oberflächenbereich ringförmig umgibt. Hierdurch kann der erste Oberflächenbereich besonders zuverlässig davor geschützt werden, dass Zellen auf ihn wandern. Mit dem erfindungsgemäßen Ansatz des steifigkeitsmodifizierten ophthalmologischen Implantats kann die Form des Linsenrandes unabhängig gestaltet werden, was die Implementierung alternativer Formen ermöglicht, wie z. B. runde Ränder, von denen bekannt ist, dass sie Dysphotopsien reduzieren. Alternativ oder zusätzlich ist es vorgesehen, dass sich der zweite Oberflächenbereich vom ersten Oberflächenbereich weg erstreckt. Hierdurch können Zellen vom ersten Oberflächenbereich weggeleitet bzw. auf dem zweiten Oberflächenbereich "gefangen" werden.

In einer weiteren vorteilhaften Ausgestaltung der Erfindung ist vorgesehen, dass der erste Oberflächenbereich und/oder der zweite Oberflächenbereich einen Elastizitätsmodulgradienten aufweist. Mit anderen Worten ist es erfindungsgemäß vorgesehen, dass der erste und/oder der zweite Oberflächenbereich keinen konstanten E-Modul, sondern einen Elastizitätsmodulgradienten aufweist bzw. aufweisen. Der Elastizitätsmodulgradient kann dabei entlang wenigstens einer Erstreckungsrichtung linear verlaufen, wodurch Zelle in eine bestimmte Richtung geleitet werden können. Ebenso kann der Elastizitätsmodulgradient entlang wenigstens einer Erstreckungsrichtung nicht-linear ausgebildet sein, um lokale Barrieren oder "Fallen" für die Zellen auszubilden, so dass durch den Steifigkeitsgradienten die bevorzugte Richtung der Zellwanderung gesteuert werden kann.

Um eine besonders wirksame Beeinflussung der Zellwanderung zu erzielen, hat es sich als vorteilhaft gezeigt, wenn der erste Oberflächenbereich und/oder der zweite Oberflächenbereich eine Dicke von mindestens 5 µm, insbesondere von mindestens 10 µm besitzt. Grundsätzlich ist es ausreichend, dass lediglich die Oberfläche des Grundkörpers die zwei oder mehr Oberflächenbereiche mit den unterschiedlichen E-Moduln aufweist. Mit anderen Worten kann das Material unterhalb der wenigstens zwei Oberflächenbereiche ein Material mit einem einheitlichen E-Modul sein. Indem die wenigstens zwei Oberflächenbereiche wenigstens 5 µm und vorzugsweise wenigstens 10 µm dick sind, ist besonders zuverlässig sichergestellt, dass die unterschiedlichen E-Moduln dauerhaft die gewünschte Wirkung auf die Zelladhäsion und -wanderung zeigen. Alternativ oder zusätzlich ist es vorgesehen, dass der erste Young'sche Modul und/oder der zweite Young'sche Modul bei 37 °C zwischen 1 kPa und 100 kPa, also beispielsweise 20 kPa, 21 kPa, 22 kPa, 23 kPa, 24 kPa, 25 kPa, 26 kPa, 27 kPa, 28 kPa, 29 kPa, 30 kPa, 31 kPa, 32 kPa, 33 kPa, 34 kPa, 35 kPa, 36 kPa, 37 kPa, 38 kPa, 39 kPa, 40 kPa oder mehr beträgt. Hierdurch werden vorteilhafte Oberflächenelastizitäten erreiche, die eine besonders zuverlässige Beeinflussung der Zelladhäsion und -wanderung erlauben. Weiterhin kann es vorgesehen sein, dass der erste Young'sche Modul und der zweite Young'sche Modul sich bei 37 °C um mindestens 10 kPa, also beispielsweise um 10 kPa, 11 kPa, 12 kPa, 13 kPa, 14 kPa, 15 kPa, 16 kPa, 17 kPa, 18 kPa, 19 kPa, 20 kPa, 21 kPa, 22 kPa, 23 kPa, 24 kPa, 25 kPa, 26 kPa, 27 kPa, 28 kPa, 29 kPa, 30 kPa, 31 kPa, 32 kPa, 33 kPa, 34 kPa, 35 kPa, 36 kPa, 37 kPa, 38 kPa, 39 kPa, 40 kPa oder mehr unterscheiden. Hierdurch wird bei Körperkerntemperatur ein ausreichender Steifigkeitsunterschied sichergestellt, um die Zelladhäsion und -wanderung gezielt zu beeinflussen.

In einer weiteren vorteilhaften Ausgestaltung der Erfindung ist vorgesehen, dass der Elastizitätsmodul des Implantats entlang einer Hauptachse des Implantats zumindest bereichsweise stetig und/oder unstetig variiert. Auch dies stellt ein geeignetes Mittel zur gezielten Beeinflussung der Zelladhäsion und -wanderung dar. Dabei können beispielsweise durch einen oder mehrere Elastizitätssprünge, die stetig oder unstetig sein können, gezielt Barrieren und/oder "Fallen" für Zellen realisiert werden. Durch kontinuierliche Variationen können entsprechende "Leitwege" für die Zellwanderung geschaffen werden.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind. Es sind auch Ausführungen und Merkmalskombinationen als offenbart anzusehen, die somit nicht alle Merkmale eines ursprünglich formulierten unabhängigen Anspruchs aufweisen. Es sind darüber hinaus Ausführungen und Merkmalskombinationen, insbesondere durch die oben dargelegten Ausführungen, als offenbart anzusehen, die über die in den Rückbezügen der Ansprüche dargelegten Merkmalskombinationen hinausgehen oder von diesen abweichen. Dabei zeigt:
- Fig. 1: eine schematische Aufsicht eines ophthalmologischen Implantats gemäß einem Ausführungsbeispiel der Erfindung;
- Fig. 2: eine schematische Aufsicht des ophthalmologischen Implantats gemäß einem weiteren Ausführungsbeispiel der Erfindung;
- Fig. 3: eine schematische Aufsicht des ophthalmologischen Implantats gemäß einem weiteren Ausführungsbeispiel der Erfindung;
- Fig. 4: eine schematische Aufsicht des ophthalmologischen Implantats gemäß einem nicht-erfindungsgemäßen Ausführungsbeispiel;
- Fig. 5: eine schematische Aufsicht des ophthalmologischen Implantats gemäß einem nicht-erfindungsgemäßen Ausführungsbeispiel;
- Fig. 6: eine schematische Seitenansicht des ophthalmologischen Implantats gemäß dem nicht-erfindungsgemäßen Ausführungsbeispiel der Fig. 4 oder 5;
- Fig. 7: eine schematische Aufsicht des ophthalmologischen Implantats gemäß einem weiteren nicht-erfindungsgemäßen Ausführungsbeispiel;
- Fig. 8: eine schematische Aufsicht des ophthalmologischen Implantats gemäß einem weiteren nicht-erfindungsgemäßen Ausführungsbeispiel;
- Fig. 9: eine schematische Seitenansicht des ophthalmologischen Implantats gemäß dem nicht-erfindungsgemäßen Ausführungsbeispiel der Fig. 7 oder 8;
- Fig. 10 bis Fig. 13: jeweils einen schematischen Längsschnitt unterschiedlicher nichterfindungsgemäßer Ausführungsbeispiele des ophthalmologischen Implantats;
- Fig. 14: einen schematischen Längsschnitt des ophthalmologischen Implantats gemäß einem weiteren Ausführungsbeispiel;
- Fig. 15: eine schematische Darstellung einer Ausführungsform eines Herstellungsprozesses eines Acrylmaterialstabes für das ophthalmologische Implantat;
- Fig. 16: eine schematische Darstellung eines alternativen oder zusätzlichen Herstellungsschritts;
- Fig. 17: eine schematische Aufsicht des ophthalmologischen Implantats gemäß einem weiteren Ausführungsbeispiel der Erfindung mit einem Diagramm radialer Steifigkeitsverläufe;
- Fig. 18: eine schematische Aufsicht des ophthalmologischen Implantats gemäß einem weiteren Ausführungsbeispiel der Erfindung mit einem Diagramm radialer Steifigkeitsverläufe; und
- Fig. 19: ein Diagramm alternativer radialer Steifigkeitsverläufe.

### Bevorzugte Ausführung der Erfindung

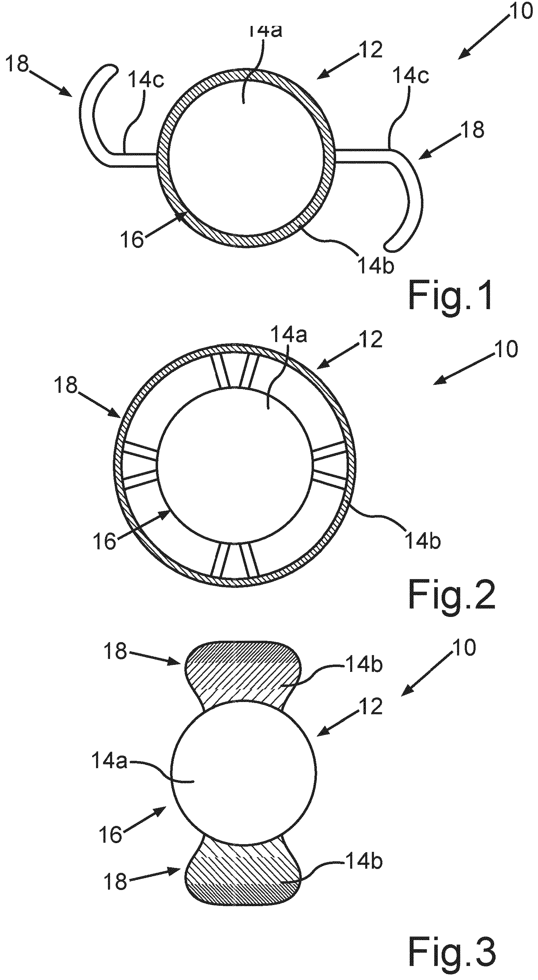

Fig. 1 zeigt eine schematische Aufsicht eines als Intraokularlinse (IOL) ausgebildeten ophthalmologischen Implantats 10 gemäß einem Ausführungsbeispiel der Erfindung. Das ophthalmologische Implantat 10 weist einen Grundkörper 12 auf, welcher einen ersten Oberflächenbereich 14a mit einem ersten Young'schen Modul Eₐ (E-Modul) und einen zweiten Oberflächenbereich 14b mit einem zweiten Young'schen Modul E_{b} umfasst, wobei sich der erste und der zweite Young'scher Modul Eₐ, E_{b} voneinander unterscheiden. Man erkennt, dass der zweite Oberflächenbereich 14b den ersten Oberflächenbereich 14a (kreis)ringförmig (360 °) umgibt. Der erste Oberflächenbereich 14a bildet dabei einen optischen Teil 16 der IOL, der von dem steifigkeitsmodifizierten, kreisringförmigen zweiten Oberflächenbereich 14b umgeben ist. Als grundsätzlich optionale haptische Teile 18 grenzen zwei hakenförmige dritte Oberflächenbereiche 14c an den zweiten Oberflächenbereich 14b an. Die dritten Oberflächenbereiche 14c können den gleichen E-Modul Eₐ wie der erste Oberflächenbereich 14a oder wie der zweite Oberflächenbereich 14b oder einen abweichenden E-Modul E_{c} besitzen.

Der zweite Oberflächenbereich 14b kann in Kombination mit oder als Ersatz für das an sich bekannte "Squared-Edge-Design" verwendet werden. Entlang dieses durch den zweiten Oberflächenbereich 14b gebildeten Randes sind die mechanischen Eigenschaften der IOL 10 so gestaltet, dass sie ein biomechanisch ungünstiges Substrat für im Auge vorkommende Zellen (LECs) bilden. Weil dieser Teil der IOL 10 aufgrund seiner Steifigkeit für die LECs mechanisch ungünstig ist, wirkt er als physikalische Barriere, die die Zellen nicht überwinden können. Diese Barriere kann, indem sie die Bewegung verhindert, das Zellwachstum auf der zentralen optischen Zone, das heißt dem ersten Oberflächenbereich 14a, der IOL 10 verhindern, was zu einer Verringerung oder zur vollständigen Vermeidung von PCO und der damit verbundenen visuellen Nebenwirkungen führt, die sich aus der direkten Anwesenheit der Zellen auf der IOL-Oberfläche ergeben. In Bezug auf die PCO-Prävention stützt sich diese Ausführungsform der IOL 10 zudem auf den Umstand, dass der Kapselsack einige Wochen nach der Implantation der IOL 10 üblicherweise schrumpft. Die LECs nutzen nach der Schrumpfung das Substrat der IOL 10 üblicherweise zur Migration, was durch die Erfindung verhindert bzw. gesteuert wird. Die Interaktion zwischen LECs mit dem steifigkeitsmodifizierten Grundkörper 12 der IOL 10 kann zudem die Migration auch auf dem Kapselsackgewebe verhindern.

Fig. 2 zeigt eine schematische Aufsicht des ophthalmologischen Implantats 10 gemäß einem weiteren Ausführungsbeispiel der Erfindung. Diese zweite Ausführungsform verhindert ebenfalls, dass LECs auf den optischen Teil 16 migrieren. Nach der Phakoemulsifikation verbleiben einige LECs in der Peripherie des Kapselsacks. Nach der Kataraktoperation proliferieren diese Zellen und wandern in Richtung des zentralen Teils der IOL 10 und des Kapselsacks, was PCO verursacht. Die hier gezeigte Ausführungsform füllt mit Hilfe des ringförmigen zweiten Oberflächenbereichs 14b den Umfang des Kapselsacks aus, so dass die restlichen Zellen nicht über diese dadurch gebildete mechanische Barriere wandern können. Der zweite Oberflächenbereich 14b bildet dabei einen über vier, gleichmäßig über den Umfang verteilten Gruppen von jeweils zwei Haptikelementen vom optischen Teil 16 beabstandeten Kapselspannring, dessen Material die erforderliche Steifigkeit hat, um die Zellmigration zu behindern. Der zweite Oberflächenbereich 14b kann dabei als eine Erweiterung des haptischen Teils 18 angesehen werden. Zumindest ein Teil der Haptik 18 wird in der äquatorialen Region des Kapselsacks im Bereich der LECs platziert. Generell können auch andere Implantattypen als Kapselspannringe vorgesehen sein. Der haptische Ring 18 kann beispielsweise auch Teil einer IOL 10 sein und/oder eine nicht-runde bzw. unregelmäßige Außenkontur besitzen.

Fig. 3 zeigt eine schematische Aufsicht des ophthalmologischen Implantats 10 gemäß einem weiteren Ausführungsbeispiel der Erfindung. Bei diesem Ausführungsbeispiel weisen die haptischen Teile 18, die zweite Oberflächenbereiche 14b bilden, welche an gegenüberliegenden Seiten des optischen Teils 16, welcher den ersten Oberflächenbereich 14a mit dem ersten E-Modul Eₐ bildet, einen Elastizitätsmodulgradienten E_{b} auf, so dass die Migration der Zellen bevorzugt vom optischen Teil 16 der IOL 10 zu den Haptiken 18 erfolgt. In diesem Fall sind die Materialeigenschaften so eingestellt, dass die Zelladhäsion am optischen Teil 16 aufgrund der elastischen Eigenschaften des ersten Oberflächenbereichs 14a behindert ist, während sie an den haptischen Teilen 18 zur Peripherie hin zunehmend stabiler wird, wo die Materialsteifigkeit bzw. der E-Modul E_{b} schließlich eine stabile Zelladhäsion begünstigt. Es hat sich gezeigt, dass eine solche Situation die Zellen dazu veranlasst, sich aus dem instabilen Adhäsionsbereich in Richtung einer Zone zu bewegen, in der sie sich stabil an den Grundkörper 12 anlagern können.

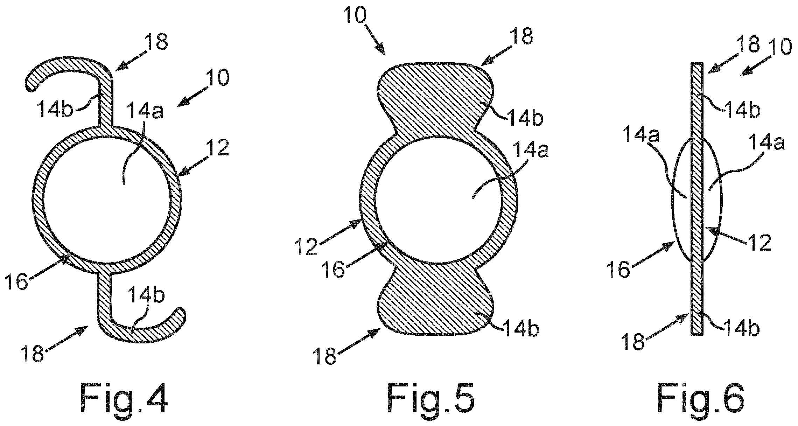

Fig. 4 und Fig. 5 zeigen schematische Aufsichten des ophthalmologischen Implantats 10 gemäß nicht-erfindungsgemäßen Ausführungsbeispielen, während Fig. 6 eine schematische Seitenansicht gemäß dem nicht-erfindungsgemäßen Ausführungsbeispiel der Fig. 4 oder 5 des ophthalmologischen Implantats 10 zeigt. Die unterschiedlichen Elastizitätsmoduln Eₐ, E_{b} der ersten und zweiten Oberflächenbereiche 14a, 14b, das heißt die unterschiedlichen Steifigkeitsbereiche des Grundkörpers 12, sind mit unterschiedlichen Füllmustern gekennzeichnet. In Fig. 4 bildet der zweite Oberflächenbereich 14b sowohl eine ringförmige Umrandung des ersten Oberflächenbereichs 14a als auch die hakenförmigen haptischen Teile 18 der IOL 10. In Fig. 5 bildet der zweite Oberflächenbereich 14b demgegenüber flügelförmige haptische Teile 18 an entgegengesetzten Seiten des optischen Teils 16. In Fig. 6 erkennt man, dass das Material des zweiten Oberflächenbereichs 14b, das den E-Modul E_{b} besitzt, einen Kern des Grundkörpers 12 bildet. Um den Mittelbereich dieses Kernmaterials, welches den optischen Teil 16 der IOL 10 bildet, ist das Material, das den ersten Oberflächenbereich 14a bildet und den E-Modul Eₐ besitzt, aufgebracht.

Das Verhältnis zwischen den Steifigkeitsunterschieden bzw. den Young'schen Moduln Eₐ, E_{b} des ersten und zweiten Oberflächenbereichs 14a, 14b hängt unter anderem von den betrachteten Zelltypen ab. Typische Steifigkeitswerte der Young'schen Moduln Eₐ, E_{b} liegen im Bereich zwischen etwa 1 kPa bis zu etwa 100 kPa. Die Steifigkeitswerte der Young'schen Moduln Eₐ, E_{b} unterscheiden sich vorzugsweise um mindestens 5 kPa, insbesondere um mindestens 10 kPa.

Die unterschiedlichen Young'schen Moduln Eₐ, E_{b} können nicht-erfindungsgemäß durch die Verwendung verschiedener Materialien in Verbindung mit der speziellen Formgebung realisiert werden. Die nicht-erfindungsgemäße Ausführungsform gemäß Fig. 4 stellt eine IOL 10 dar, die aus zwei unterschiedlichen Materialien besteht. Das zweite Material, welches den zweiten Oberflächenbereich 14b bildet, ist hydrophil und im Vergleich zum ersten, hydrophoben Material, das den ersten Oberflächenbereich 14a bildet, weicher bzw. elastischer. Das zweite Material bildet mit anderen Worten die weniger steife Haptik 18 und einen Ring um den steiferen optischen Teil 16. Das zweite Material wirkt dabei wie eine physikalische Barriere und verhindert die Zellmigration.

Bei allen nicht-erfindungsgemäßen Ausführungsformen gemäß Fig. 4 und Fig. 5 kann der Ring des haptischen Teils 18 um den optischen Teil 16 relativ erhöht sein bzw. eine größere Querschnittsdicke aufweisen. Hierdurch können zwei physikalische Barrieren kombiniert werden, eine erste der Materialsteifigkeit und eine zweite der Formgebung.

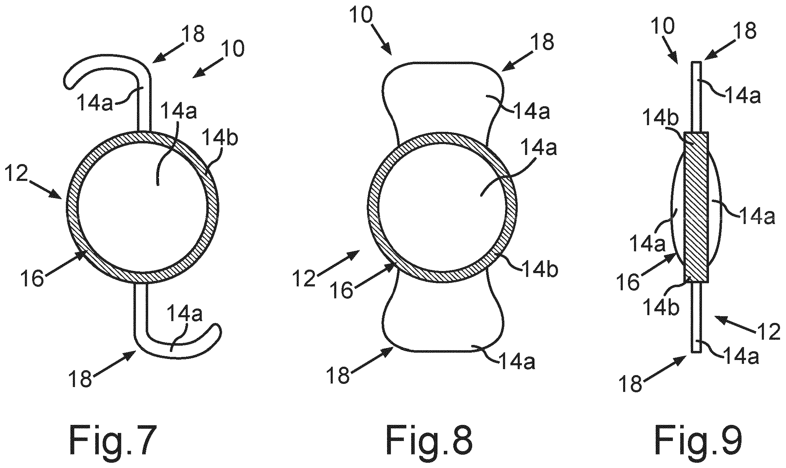

Fig. 7 zeigt eine schematische Aufsicht des ophthalmologischen Implantats 10 gemäß einem weiteren nicht-erfindungsgemäßen Ausführungsbeispiel der Erfindung. Der generelle Aufbau ähnelt dem in Fig. 4 gezeigten Ausführungsbeispiel, wobei im Unterschied hierzu die hakenförmigen haptischen Teile 18 vorliegend ebenfalls aus dem gleichen Material gebildet sind, das den ersten Oberflächenbereich 14a bildet bzw. den ersten E-Modul Eₐ besitzt.

Gleiches gilt für das in Fig. 8 gezeigte nicht-erfindungsgemäße Ausführungsbeispiel, bei dem im Unterschied zum in Fig. 5 gezeigten Ausführungsbeispiel die flügelförmigen haptischen Teile 18 vorliegend ebenfalls aus dem gleichen Material gebildet sind, das den ersten Oberflächenbereich 14a bildet bzw. den ersten E-Modul Eₐ besitzt.

Fig. 9 zeigt eine schematische Seitenansicht des ophthalmologischen Implantats 10 gemäß dem nicht-erfindungsgemäßen Ausführungsbeispiel der Fig. 7 oder Fig. 8. Im Unterschied zum in Fig. 6 gezeigten Ausführungsbeispiel erstreckt sich das den Kern des Grundkörpers 12 bildende zweite Material, das den zweiten E-Modul E_{b} besitzt und den zweiten Oberflächenbereich 14b bildet, vorliegend nicht über die gesamte Länge des Implantats 10. Stattdessen schließt sich an den Rändern des zweiten Materials nochmals das erste Material an, das den ersten E-Modul Eₐ besitzt und den ersten Oberflächenbereich 14a bildet. Darüber hinaus weist das zweite Material eine größere Dicke auf als im in Fig. 6 gezeigten Beispiel.

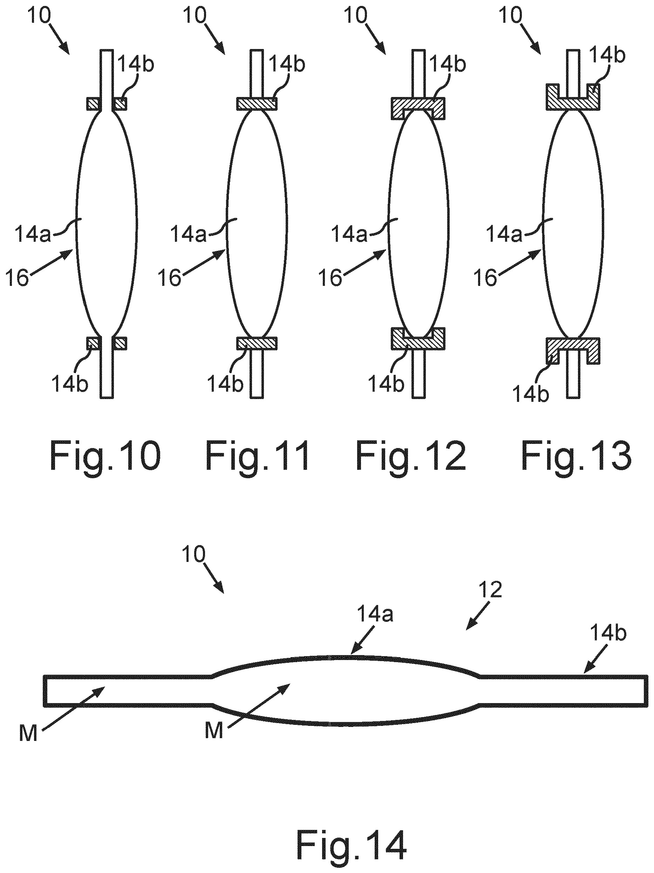

Fig. 10 bis Fig. 13 zeigen schematische Längsschnitte unterschiedlicher nichterfindungsgemäßer Ausführungsbeispiele des ophthalmologischen Implantats 10. Man erkennt, dass das zweite Material, das den zweiten E-Modul E_{b} besitzt und den zweiten Oberflächenbereich 14b bildet, (Kreis)Ringe mit unterschiedlichen Querschnittsgeometrien um den optischen Teil 16, der aus dem ersten Material mit dem ersten E-Modul Eₐ besteht, bildet. Der zweite Oberflächenbereich 14b ist dabei immer erhaben gegenüber dem ersten Oberflächenbereich 14a und erzeugt quasi zwei mechanische Barrieren, eine erste durch die geänderte Materialsteifigkeit und eine zweite durch die Formgebung der Ringe. Man sieht, dass dieser Ansatz der lokalen Steifigkeitsvariierung bzw. Elastizitätseinstellung generell nicht nur auf eine Seite des Implantats 10 beschränkt sein kann, sondern dass die unterschiedlichen Materialeigenschaften sowohl auf der vorderen als auch auf der hinteren Seite von Implantaten 10 verwendet werden können, was eine besonders zuverlässige PCO-Prävention ermöglicht. Dies steht im Gegensatz zum aus dem Stand der Technik bekannten Ansatz des "Square-Edge-Designs", das nur auf der posterioren, aber nicht auf der anterioren Seite der IOL 10 vorhanden ist.

Fig. 14 zeigt einen schematischen Längsschnitt des erfindungsgemäßen ophthalmologischen Implantats 10 gemäß einem weiteren Ausführungsbeispiel. Man erkennt, dass das Implantat 10 bzw. sein Grundkörper 12 im Inneren aus ein und demselben Material M besteht, welches lediglich lokal oberflächlich modifiziert ist, um die unterschiedlichen Oberflächenbereiche 14a, 14b mit den unterschiedlichen Young'schen Moduln Eₐ, E_{b} zu erzeugen. Die Oberflächenbereiche 14a, 14b mit veränderter Steifigkeit können durchgehend sein bzw. die gesamte Oberfläche der IOL 10 bilden oder nur in bestimmten Oberflächenbereichen des Grundkörpers 12 vorhanden sein. Gegebenenfalls kann bereichsweise ein dritter oder weiterer Young'scher Modul E_{c} gegeben sein. Die Schichtdicke der Oberflächenbereiche 14a, 14b beträgt generell vorzugsweise mindestens 10 µm.

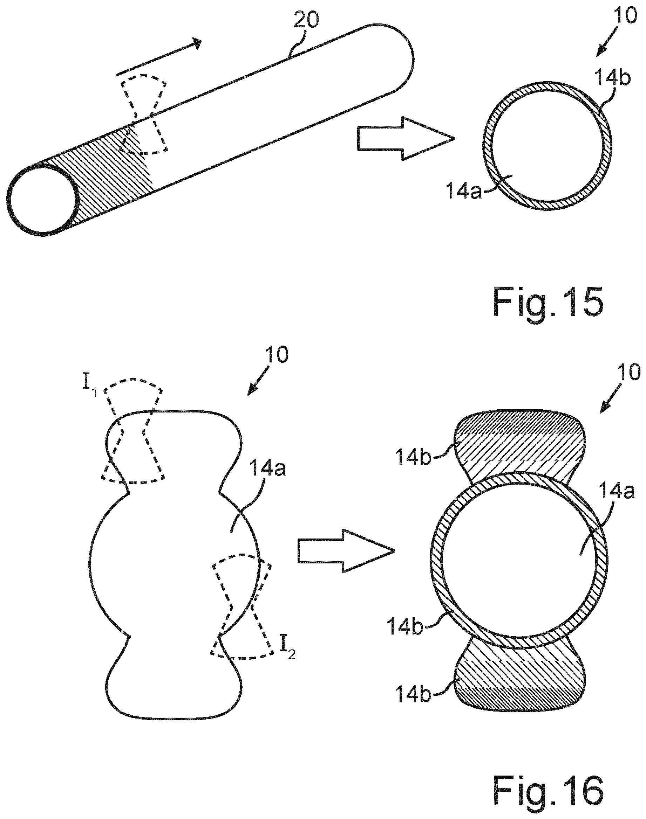

Fig. 15 zeigt eine schematische Darstellung einer Ausführungsform eines Herstellungsprozesses eines beispielhaft aus einem substituierten oder unsubstituierten (Meth)Acrylmaterial bestehenden Stabes 20 für das ophthalmologische Implantat 10. Eine Kontrolle über die Steifigkeit bzw. der unterschiedlichen Young'schen Moduln Eₐ, E_{b} kann dabei bereits auf der Stufe des Bulkmaterials M erreicht werden, indem beispielsweise Stäbe 20 aus einem (Meth)Acrylpolymer einer Strahlung (z. B. Laser- bzw. UV-Strahlen) ausgesetzt werden, so dass in den bestrahlten Bereichen (mehr) chemischen Bindungen gebildet werden, die das Polymer vernetzen bzw. stärker vernetzen. Anschließend kann der Stab 20 in einzelne Scheiben zerteilt werden, wodurch Implantate 10 mit einem ringförmigen zweiten Oberflächenbereich 14b und einem kreisförmigen ersten Oberflächenbereich 14a hergestellt werden können. Alternativ oder zusätzlich kann während der Polymerisation des Stabs 20 eine unterschiedliche Menge eines oder mehrerer Vernetzungsmittel zugegeben werden, so dass der Stab 20 aus verschiedenen Schichten mit dem jeweils gewünschten Vernetzungsgraden und damit aus Schichten mit jeweils unterschiedlichen Young'schen Moduln Eₓ hergestellt werden kann. Solche Stäbe 20 können anschließend wieder in Scheiben geschnitten und zu Linsen verarbeitet werden.

Fig. 16 zeigt eine schematische Darstellung eines alternativen oder zusätzlichen Herstellungsschritts. Hierbei können die Modifikationen des Young'schen Moduls direkt auf dem bereits hergestellten Implantat 10 erzeugt werden, beispielsweise durch Bestrahlung mit energiereichen Strahlen I₁, I₂ und/oder durch Beaufschlagung der Oberfläche mit reaktiven Materialien, beispielsweise mit Sauerstoffradikalen (Sauerstoffplasma). Dies stellt auch eine technisch einfache Möglichkeit zum Erzeugen von zumindest oberflächlichen Elastizitätsmodulgradienten dar.

Generell kann die Steifigkeit von Materialien (zum Beispiel Kollagen, Poly(meth)acrylamid etc.) durch den Grad der Vernetzung zwischen den Polymerketten maßgeschneidert werden. Als allgemeine Regel gilt dabei generell, dass die Materialmatrix umso leichter verformbar ist, je geringer die Anzahl der Vernetzungen zwischen den Polymerketten ist, wodurch das Material entsprechend weicher wird bzw. einen niedrigeren Young'schen Modul E besitzt. Die Kontrolle über die Vernetzung kann chemisch und/oder durch Strahlungseinwirkung erreicht werden. Die Steifigkeit einiger Materialien, wie z. B. Polyacrylamid, lässt sich chemisch einstellen, indem man die Art und/oder Menge des Vernetzungsmittels, das während des chemischen Reaktionsprozesses, der zur Bildung der Polymermatrix führt, zugegeben wird, und/oder das Aushärtungsverfahren anpasst. In ähnlicher Weise kann die Bestrahlung mit UV-(Vis)-Strahlung neue Bindungen im Polymernetzwerk erzeugen und den Quervernetzungsgrad erhöhen. Damit ist eine Kontrolle und Einstellung der lokalen Oberflächeneigenschaften, insbesondere der lokalen E-Moduln, von polymeren Materialien, die für die erfindungsgemäßen ophthalmologischen Implantate 10 eingesetzt werden können, möglich.

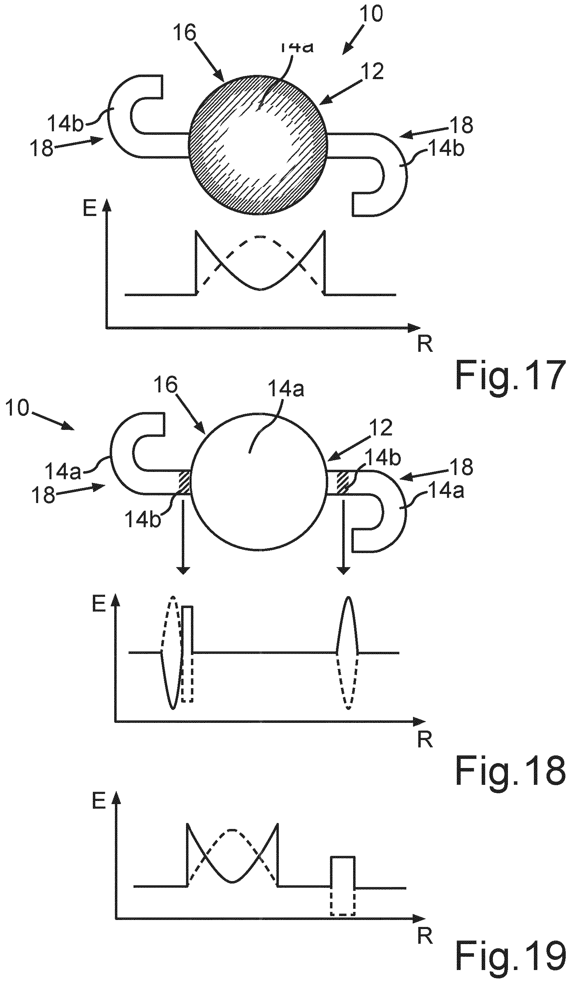

Fig. 17 eine schematische Aufsicht des ophthalmologischen Implantats 10 gemäß einem weiteren Ausführungsbeispiel der Erfindung zusammen mit einem schematischen Diagramm, in welchem auf der Ordinate der lokale E-Modul E (in kPa) und auf der Abszisse die radiale Position R (in mm) entlang einer Hauptachse des Implantats 10 angegeben ist. Dabei stellen der E-Modulverlauf mit der durchgezogenen Linie und der der E-Modulverlauf mit der gestrichelten Linie zwei beispielhafte, alternative E-Modulnverläufe dar. Wie bereits ausgeführt sollte der Steifigkeitsgradient so gestaltet sein, dass die Zellen bevorzugt von der Mitte des Grundkörpers 12 weg radial nach außen wandern. Die Steifigkeit der haptischen Teile 18 mit den zweiten Oberflächenbereichen 14b bzw. den zweiten E-Moduln E_{b} ist so ausgelegt, dass sie als Barriere für die Zellwanderung wirken, indem sie deren Bewegung verlangsamen oder "Fallen" bzw. "Senken" bilden, in denen die Zellen bevorzugt konzentriert werden und aus denen sie nicht mehr heraus wandern können. Dementsprechend weist der erste Oberflächenbereich 14a und damit der optische Teil 16 in einem Ausführungsbeispiel einen mit der gestrichelten Linie gezeigten Elastizitätsmodulgradienten auf, der ausgehend von einem Rand des optischen Teils 16 bis zur Mitte der IOL 10 hin kontinuierlich ansteigt und anschließend zum anderen Rand hin kontinuierlich wieder auf das Niveau des zweiten Oberflächenbereichs 14b abfällt. Alternativ weist der erste Oberflächenbereich 14a einen mit der durchgezogenen Linie gezeigten Elastizitätsmodulgradienten auf, der ausgehend von einem Rand des optischen Teils 16 zunächst senkrecht bzw. diskontinuierlich ansteigt, zur Mitte der IOL 10 hin kontinuierlich abfällt und anschließend zum anderen Rand hin kontinuierlich wieder ansteigt, wo der E-Modul Eₐ wieder senkrecht bzw. diskontinuierlich auf das Niveau des zweiten Oberflächenbereichs 14b abfällt. Anstelle der beschriebenen Senke kann es auch vorgesehen sein, dass der E-Modul Eₐ des ersten Oberflächenbereichs 14a konstant ist und damit eine Art Plateau bezüglich des radialen Elastizitätsverlaufs bildet.

Fig. 18 zeigt eine schematische Aufsicht des ophthalmologischen Implantats 10 gemäß einem weiteren Ausführungsbeispiel der Erfindung mit einem schematischen Diagramm radialer Steifigkeitsverläufe. Erneut sind in Diagramm auf der Ordinate der lokale E-Modul E (in kPa) und auf der Abszisse die radiale Position R (in mm) entlang einer Hauptachse des Implantats 10 angegeben. Man erkennt, dass, je nachdem, ob der Elastizitätsmodulverlauf der durchgezogenen oder der gestrichelten Linie folgt, an unterschiedlichen radialen Positionen des Implantats 10 "Fallen" bzw. "Senken" für Zellen (niedrigerer E-Modul) oder "Barrieren" (höherer E-Modul) gegen Zellmigration gebildet sein können.

Fig. 19 zeigt ein Diagramm alternativer radialer Steifigkeitsverläufe. Man erkennt wie im vorherigen Beispiel, dass unterschiedliche E-Modulverläufe entlang der Oberfläche des Implantats 10 möglich sind und dass wahlweise "Barrieren", das heißt relative lokale E-Modul-Erhöhungen, und/oder "Senken", das heißt relative lokale E-Modul-Erniedrigungen, vorgesehen sein können, um Zellen in einer gewünschten Art und Weise auf der Oberfläche des Implantats 10 zu lenken.

Mit Hilfe der erfindungsgemäßen ophthalmologischen Implantate 10 kann also die Migration von Linsenepithelzellen kontrolliert werden, indem die Steifigkeit bzw. der Young'sche Modul E des Grundkörpers 12 zumindest oberflächlich so verändert wird, dass die Migration in die optische Zone bzw. den optischen Teil 16 verhindert wird und/oder dass die Zellen gezielt in vorher festgelegte Bereiche des Implantats 10 gelenkt werden, wo ihre eventuelle Proliferation keine Sehstörungen oder anderweitige Probleme verursacht. Die Steifigkeitsmodifikation wird erfindungsgemäß auf die Oberfläche des Grundkörpers 12 beschränkt angewendet, solange der lokale Steifigkeitswert (E-Modul) und die geometrische Verteilung, beispielsweise durch unterschiedliche Quervernetzungsgrade des IOL-Polymermaterials, ausreichend genau deterministisch eingestellt werden können.

Weitere nicht-erfindungsgemäße Ausführungsformen der Erfindung beruhen auf der Kombination verschiedener Materialien M mit unterschiedlichen elastischen Eigenschaften zur Herstellung des Implantats 10 bzw. des Grundkörpers 12. Beispielsweise können hydrophobe und hydrophile (Meth)Acrylmaterialien kombiniert werden, um ein Hybrid-Implantat 10 (IOL) zu schaffen. Bei einem solchen Implantat 10 kann beispielsweise ähnlich den vorherigen Ausführungsbeispielen in Fig. 1 bis Fig. 9 ein weicherer bzw. weniger steifer Oberflächenbereich 14b aus einem hydrophilen Material bestehen, das als Barriere gegen die Zellmigration in Richtung der klaren optischen Zone 16 aus einem steiferen, hydrophoben Material mit dem ersten Oberflächenbereich 14a wirken kann. Das zweite Material mit dem zweiten Young'schen Modul kann auch nur an den in den vorstehenden Ausführungsform gezeigten Bereichen als Beschichtung vorgesehen sein, da die Zellen auf diese unterschiedlichen Oberflächenelastizitäten reagieren, selbst wenn die Dicke der Beschichtung nur 5 bis 10 µm dick ist.

Durch die erfindungsgemäßen Implantate 10 kann die Notwendigkeit einer scharfen Kante bei IOLs als Präventivmaßnahme gegen PCO entfallen. Dies ermöglicht einen einfacheren Zuschnitt des Linsenprofils, wodurch zusätzlich abweichende Randgestaltungen möglich sind, die z.B. Dysphotopsien vermeiden helfen. Darüber hinaus können die erfindungsgemäßen Implantate 10 die Laser-Kapsulotomie obsolet machen.

Die in den Unterlagen angegebenen Parameterwerte zur Definition von Prozess- und Messbedingungen für die Charakterisierung von spezifischen Eigenschaften des Erfindungsgegenstands sind auch im Rahmen von Abweichungen - beispielsweise aufgrund von Messfehlern, Systemfehlern, DIN-Toleranzen und dergleichen - als vom Rahmen der Erfindung mitumfasst anzusehen. Generell sind physikalische Eigenschaften und insbesondere die im Rahmen der vorliegenden Offenbarung genannten Steifigkeitswerte bzw. die E-Moduln bei 37 °C und Normaldruck (1,013 bar) zu ermitteln.

### Bezugszeichenliste

- 10: Implantat
- 12: Grundkörper
- 14a: erster Oberflächenbereich
- 14b: zweiter Oberflächenbereich
- 14c: dritter Oberflächenbereiche
- 16: optischer Teil
- 18: haptischer Teil
- 20: Stab
- Eₐ: erster Young'scher Modul
- E_{b}: zweiter Young'scher Modul
- M: Material
- I_{1, 2}: Strahlung

## Patentansprüche

1. Ophthalmologisches Implantat (10) mit einem Grundkörper (12), welcher zumindest einen ersten Oberflächenbereich (14a) mit einem ersten Young'schen Modul (Eₐ) und einen zweiten Oberflächenbereich (14b) mit einem zweiten Young'schen Modul (E_{b}) umfasst, wobei sich der erste und der zweite Young'scher Modul (Eₐ, E_{b}) voneinander unterscheiden, wobei der Grundkörper (12) wenigstens einen haptischen Teil (18) und wenigstens einen optischen Teil (16) umfasst, wobei der optische Teil (16) den ersten Oberflächenbereich (14a) und der haptische Teil (18) den zweiten Oberflächenbereich (14b) umfasst, **dadurch gekennzeichnet, dass**
der Grundkörper (12) zumindest überwiegend aus einem einzelnen Material (M) besteht, das im ersten und/oder im zweiten Oberflächenbereich (14a, 14b) modifiziert, insbesondere unterschiedlich stark quervernetzt ist, um die unterschiedlichen Young'schen Moduln (Eₐ, E_{b}) zu erzeugen.

2. Ophthalmologisches Implantat (10) nach Anspruch 1,
**dadurch gekennzeichnet, dass**
der zweite Oberflächenbereich (14b) unmittelbar an den ersten Oberflächenbereich (14a) angrenzt und/oder dass der zweite Oberflächenbereich (14b) vom ersten Oberflächenbereich (14a) beabstandet angeordnet ist und/oder dass der zweite Oberflächenbereich (14b) den ersten Oberflächenbereich (14a) ringförmig umgibt und/oder dass sich der zweite Oberflächenbereich (14b) vom ersten Oberflächenbereich (14a) weg erstreckt.

3. Ophthalmologisches Implantat (10) nach Anspruch 1 oder 2,
**dadurch gekennzeichnet, dass**
der erste Oberflächenbereich (14a) und/oder der zweite Oberflächenbereich (14b) einen Elastizitätsmodulgradienten (Eₐ, E_{b}) aufweist.

4. Ophthalmologisches Implantat (10) nach einem der Ansprüche 1 bis 3,
**dadurch gekennzeichnet, dass**
der erste Oberflächenbereich (14a) und/oder der zweite Oberflächenbereich (14b) eine Dicke von mindestens 5 µm, insbesondere von mindestens 10 µm besitzt und/oder dass der erste Young'sche Modul (Eₐ) und/oder der zweite Young'sche Modul (E_{b}) bei 37 °C zwischen 1 kPa und 100 kPa, insbesondere zwischen 20 kPa und 40 kPa beträgt und/oder dass sich der erste Young'sche Modul (Eₐ) und der zweite Young'sche Modul (E_{b}) bei 37 °C um mindestens 10 kPa unterscheiden.

5. Ophthalmologisches Implantat (10) nach einem der Ansprüche 1 bis 4,
**dadurch gekennzeichnet, dass**
der Elastizitätsmodul (E) des Implantats (10) entlang einer Hauptachse des Implantats (10) zumindest bereichsweise stetig und/oder unstetig variiert.

## Claims

1. Ophthalmological implant (10) having a main body (12) which comprises at least a first surface region (14a) with a first Young's modulus (Eₐ) and a second surface region (14b) with a second Young's modulus (E_{b}), wherein the first and second Young's moduli (Eₐ, E_{b}) differ from each other, wherein the main body (12) comprises at least one haptic part (18) and at least one optical part (16), wherein the optical part (16) comprises the first surface region (14a), and the haptic part (18) comprises the second surface region (14b), **characterized in that**
the main body (12) at least predominantly consists of a single material (M), which is modified in the first and/or in the second surface region (14a, 14b), in particular crosslinked to different degrees, in order to generate the different Young's moduli (Eₐ, E_{b}).

2. Ophthalmological implant (10) according to Claim 1, **characterized in that**
the second surface region (14b) is directly adjacent to the first surface region (14a) and/or **in that** the second surface region (14b) is arranged at a distance from the first surface region (14a) and/or **in that** the second surface region (14b) surrounds the first surface region (14a) in ring-shaped fashion and/or **in that** the second surface region (14b) extends away from the first surface region (14a).

3. Ophthalmological implant (10) according to Claim 1 or 2,
**characterized in that**
the first surface region (14a) and/or the second surface region (14b) has a modulus of elasticity gradient (Eₐ, E_{b}).

4. Ophthalmological implant (10) according to any of Claims 1 to 3,
**characterized in that**
the first surface region (14a) and/or the second surface region (14b) has a thickness of at least 5 µm, in particular at least 10 µm, and/or **in that** the first Young's modulus (Eₐ) and/or the second Young's modulus (E_{b}) is between 1 kPa and 100 kPa, in particular between 20 kPa and 40 kPa, at 37°C and/or **in that** the first Young's modulus (Eₐ) and the second Young's modulus (E_{b}) differ by at least 10 kPa at 37°C.

5. Ophthalmological implant (10) according to any of Claims 1 to 4,
**characterized in that**
the modulus of elasticity (E) of the implant (10) along a main axis of the implant (10) varies continuously and/or discontinuously at least in some regions.

## Revendications

1. Implant ophtalmologique (10) présentant un corps de base (12), qui comprend au moins une première zone surfacique (14a) dotée d'un premier module de Young (Eₐ) et une seconde zone surfacique (14b) dotée d'un second module de Young (E_{b}), le premier et le second module de Young (Eₐ, E_{b}) se distinguant l'un de l'autre, le corps de base (12) comprenant au moins une partie haptique (18) et au moins une partie optique (16), la partie optique (16) comprenant la première zone surfacique (14a) et la partie haptique (18) comprenant la seconde zone surfacique (14b),
**caractérisé en ce que**
le corps de base (12) est constitué au moins principalement d'un seul matériau (M) qui est modifié, en particulier réticulé à différents degrés, dans la première et/ou la seconde zone surfacique (14a, 14b) afin d'obtenir les modules de Young (Eₐ, E_{b}) différents.

2. Implant ophtalmologique (10) selon la revendication 1,
**caractérisé en ce que**
la seconde zone surfacique (14b) est immédiatement adjacente à la première zone surfacique (14a) et/ou **en ce que** la seconde zone surfacique (14b) est agencée à une certaine distance de la première zone surfacique (14a) et/ou **en ce que** la seconde zone surfacique (14b) entoure la première zone surfacique (14a) de manière annulaire et/ou **en ce que** la seconde zone surfacique (14b) s'étend à l'opposé de la première zone surfacique (14a).

3. Implant ophtalmologique (10) selon la revendication 1 ou 2,
**caractérisé en ce que**
la première zone surfacique (14a) et/ou la seconde zone surfacique (14b) présente(nt) un gradient de module d'élasticité (Eₐ, E_{b}).

4. Implant ophtalmologique (10) selon l'une des revendications 1 à 3,
**caractérisé en ce que**
la première zone surfacique (14a) et/ou la seconde zone surfacique (14b) possède(nt) une épaisseur d'au moins 5 µm, en particulier d'au moins 10 µm et/ou **en ce que** le première module de Young (Eₐ) et/ou le second module de Young (E_{b}), à 37°C, est situé entre 1 kPa et 100 kPa, en particulier entre 20 kPa et 40 kPa et/ou **en ce que** le première module de Young (Eₐ) et le second module de Young (E_{b}), à 37°C, se distinguent d'au moins 10 kPa.

5. Implant ophtalmologique (10) selon l'une des revendications 1 à 4,
**caractérisé en ce que**
le module d'élasticité (E) de l'implant (10) varie en continu et/ou de manière discontinue, au moins par zones, le long d'un axe principal de l'implant (10).
